# EUROPÄISCHE PATENTSCHRIFT

(11) **EP 1 228 247 B1**
(45) Veröffentlichungstag und Bekanntmachung des Hinweises auf die Patenterteilung: **04.08.2004**
(21) Anmeldenummer: 00987122.9
(22) Anmeldetag: 12.11.2000
(51) Int. Cl.: C12Q 1/68

(54) **VERFAHREN ZUR KONTROLLIERBAREN DURCHFÜHRUNG KOMPLEXER PCR-AMPLIFIKATIONEN**
METHOD FOR THE CONTROLLED IMPLEMENTATION OF COMPLEX PCR AMPLIFICATIONS
PROCEDE POUR LA REALISATION CONTROLABLE D'AMPLIFICATIONS PAR PCR COMPLEXES

(30) Priorität: 12.11.1999 DE 19956203; 12.10.2000 DE 10051714
(43) Veröffentlichungstag der Anmeldung: 07.08.2002
(73) Patentinhaber: Epigenomics AG, 10435 Berlin (DE)
(72) Erfinder: BERLIN, Kurt, 14532 Stahnsdorf (DE)
(74) Vertreter: Schubert, Klemens, Dr.
(86) Internationale Anmeldenummer: PCT/DE2000/003973
(87) Internationale Veröffentlichungsnummer: WO 2001/036669

(56) Entgegenhaltungen:
- WO-A-93/25563
- WO-A-97/46705
- WO-A-99/28498
- CHEN JEREMY J W ET AL: "Profiling expression patterns and isolating differentially expressed genes by cDNA microarray system with colorimetry detection" GENOMICS,ACADEMIC PRESS, SAN DIEGO,US, Bd. 51, Nr. 3, 1. August 1998 (1998-08-01), Seiten 313-324, XP002159705 ISSN: 0888-7543
- REIN ET AL: "Identifying 5-methylcytosine and related modifications in DNA genomes" NUCLEIC ACIDS RESEARCH,OXFORD UNIVERSITY PRESS, SURREY,GB, Bd. 26, Nr. 10, 1998, Seiten 2255-2264, XP002143106 ISSN: 0305-1048

## Beschreibung

Die Erfindung betrifft ein Verfahren zur kontrollierbaren Durchführung komplexer PCR-Amplifikationen.

Komplexe PCR-Amplifikationen, z. B. "whole genome amplifikations", werden zur simultanen Vervielfältigung einer Vielzahl von Fragmenten aus DNA-Proben genutzt. Die erhaltenen, hochdiversen Fragmente konnen unter anderem für Genotypisierungen, Mutationsanalyse und verwandte Themenbereiche genutzt werden.

Die Verfahren sind allerdings nur schwer validierbar und kalibrierbar, eben aufgrund der Komplexität und der Tatsache, daß ein großer Teil der amplifizierten Sequenz meist unbekannt ist. Die vorliegende Erfindung befaßt sich mit komplexen PCR Amplifikationen, die mittels eines angepaßten Oligomer-Arrays einer vollständigen Analyse zugänglich sind.

PCR Reaktionen (Polymerase Kettenreaktionen) werden normalerweise mit zwei spezifisch bindenden Primern durchgeführt, wobei einer komplementär zum (+)-Strang, der andere komplementär zum (-) Strang des zu amplifizierenden Templates ist. Das Ziel einer solchen Amplifikation ist es, ein bestimmtes Fragment der Templat DNA zu vervielfältigen, das in der Regel genau definiert und in seiner Basensequenz zum großen Teil oder vollständig bekannt ist.

Es sind auch PCR Reaktionen bekannt, die mehr als zwei verschiedene Primer einsetzen. Meist dienen sie zur Amplifikation mehrerer, ebenfalls wenigstens zum großen Teil in ihrer Basensequenz bekannter Fragmente gleichzeitig in einem Gefäß. Auch in diesem Fall binden die eingesetzten Primer spezifisch an bestimmte Abschnitte der Templat DNA. Man spricht in solchen Fällen von "Multiplex-PCR", meist hat sie nur zum Ziel, mehrere Fragmente gleichzeitig amplifizieren zu können und so Material und experimentellen Aufwand einzusparen.

PCR-Reaktionen, die nicht zuvor bekannte Fragmente amplifizieren, können mit nicht spezifisch an bestimmte Regionen bindenden Primern durchgeführt werden. Diese sind dann so konzipiert, daß komplementäre Bindungsstellen in der Templat DNA statistisch betrachtet mehrmals auftreten sollten. Wir sprechen in diesem Fall von komplexen PCR Amplifikationen. Solche Amplifikationen konnen genutzt werden, um einen bestimmten Anteil beispielsweise eines Genoms statistisch verteilt zu amplifizieren. Je nach der Anzahl der möglichen Bindungsstellen eines Primers im Genom kann die Komplexität der Amplifikate reguliert werden. Die Primer können entweder sehr kurz sein, über universell paarende Basen verfügen oder aber als kombinatorische Bibliothek von Sequenzen synthetisiert worden sein. "Whole genome amplifications" werden beispielsweise in den folgenden Publikationen beschrieben: L. Zhang, et al.; Whole genome amplification from a single cell: Implications for genetic analysis; Proc. Natl. Acad. Sci. USA (1992), 89, 5847-51; K. Kristjansson, et al.; Preimplantation single cell analyses of dytrophin gene deletion using whole genome amplification; Nature Genetics (1994), 6, 19-23; P. O. Brown; Genome scanning methods, Current Opinion in Genetics and Development (1994), 4, 366-73; M. T. Barrett, et al.; Genotypic analysis of multiple loci in somatic cells by whole genome amplification; Nucl. Acids. Res. (1995), 23, 3488-92; D. Grothues, et al. PCR amplification of megabase DNA with tagged random primers (T-PCR); Nucl. Acids. Res. (1993), 21, 1321-2; J. Welsh et al.; Fingerprinting genomes using PCR with arbitrary primers; Nucl. Acids. Res. (1990), 18, 7213-8; V. G. Cheung et al.; Whole genome amplification using a degenerate oligonucleotide primer allows hundreds of genotypes to be performed on less than one nanogram of genomic DNA; Proc. Natl. Acad. Sci. USA (1996), 93, 14676-9.

Im Bereich der DNA-Chips ist ein Prinzip von allen Entwicklungen am weitesten fortgeschritten, wie dies beispielsweise in den US-A 5,593,839, US-A 5,999,695 oder US-A 5,631,734 beschrieben ist. Aber es sind auch eine Anzahl anderer DNA-Chips mit verschiedenen Eigenschaften für spezielle Anwendungen bekannt (z.B. US-A 5,667,667, US-A 5,525,464 oder US-A 5,492,806 oder z.B. Goffeau, A., Nature 385, 202-203; Weiler, J. and Hoheisel, J., Anal. Biochem. 243,218-227; Chee, M. et al., Science 274, 610-614). Jüngere Publikationen berichten schon über einen kommerziell verfügbaren HIV-Chip, der die Untersuchung des kompletten HIV-Genoms gestattet. Gegen bis zu 400.000 Oligonukleotide werden fluoreszenzmarkierte PCR-Produkte der zu untersuchenden Probe hybridisiert. Die Auswertung der Signale erfolgt mit Hilfe von CCD-Kameras oder speziellen Fluoreszenzscannern. Es wird die seit langem bekannte Fähigkeit solcher Systeme zur allelspezifischen Hybridisierung genutzt. Das bedeutet: Nur dort, wo die Probe absolut komplementär zu einem fixierten Oligonukleotid ist, bleibt am Ende der Hybridisierungsund Waschprozeduren ein Signal erhalten. Die Untersuchung einer bekannten Gensequenz auf Mutationen gelingt, weil sich nicht nur jeder Teilbereich der gesamten Sequenz in Form von Oligonukleotidsequenzen auf der Matrix befindet, sondern weil dies auch für jede mögliche Abweichung von der Normalsequenz zutrifft. Die Effizienz der Chip-Prozedur rührt zu einem Teil daher, daß mit zwei einfachen Arbeitsschritten, nämlich der Hybridisierung und dem Waschen, die Sequenzinformation einer Vielzahl von Genen oder Genorten erhalten wird.

Die Detektion von verschiedenen in einer Amplifikation eingebauten markierten Nukleotid-Analoga oder am 5'Ende markierter DNA auf einem (wie auch immer gearteten) DNA-Chip läßt sich auf verschiedenste Art bewerkstelligen. Eine Möglichkeit ist die Detektion mit einer CCD-Camera, welche Fluoreszenzsignale registriert, welche auf dem Chip die erfolgte Bindung einer fluoreszenz-markierten beliebigen Sequenz anzeigen.

5-Methylcytosin ist die häufigste kovalent modifizierte Base in der DNA eukaryotischer Zellen. Sie spielt beispielsweise eine Rolle in der Regulation der Transkription, genomischem Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüberhinaus geht bei einer PCR-Amplifikation die epigenetische Information, die die 5-Methylcytosine tragen, vollständig verloren.

Es sind mehrere Verfahren bekannt, die diese Probleme lösen. Meist wird eine chemische Reaktion oder enzymatische Behandlung der genomischen DNA durchgeführt, infolge derer sich die Cytosin von den Methylcytosin Basen unterscheiden lassen. Eine gängige Methode ist die Umsetzung von genomischer DNA mit Bisulfit, die nach alkalischer Hydrolyse in zwei Schritten zu einer Umwandlung der Cytosin Basen in Uracil führt (Shapiro, R., Cohen, B., Servis, R. Nature 227, 1047 (1970)). 5-Methylcytosin bleibt unter diesen Bedingungen unverändert. Die Umwandlung von C in U führt zu einer Veränderung der Basensequenz, aus der sich durch Sequenzierung nun die ursprünglichen 5-Methylcytosine ermitteln lassen (nur diese liefern noch eine Bande in der C-Spur).

Matrix-assistierte Laser Desorptions/Ionisations Massenspektrometrie (MALDI) ist eine neue, sehr leistungsfähige Entwicklung für die Analyse von Biomolekülen (Karas, M. and Hillenkamp, F. 1988. Laser desorption ionization of proteins with molecular masses exceeding 10.000 daltons. Anal. Chem. 60: 2299-2301). Ein Analytmolekül wird in eine im UV absorbierende Matrix eingebettet. Durch einen kurzen Laserpuls wird die Matrix ins Vakuum verdampft und das Analyt so unfragmentiert in die Gasphase befördert. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere. Die Flugzeit wird in die Masse der Ionen umgerechnet.

Technische Neuerungen der Hardware haben die Methode signifikant verbessert. Erwähnenswert ist delayed extraction (DE). Für DE wird die Beschleunigungsspannung mit einer Verzögerung zum Laserpuls eingeschaltet und dadurch eine verbesserte Auflösung der Signale erreicht, weil die Zahl der Stöße verringert wird.

Für die Abtastung eines immobilisierten DNA-Arrays sind vielfach fluoreszent markierte Sonden verwendet worden. Besonders geeignet sind für die Fiuoreszenzmarkierung ist das einfache Anbringen von Cy3 und Cy5 Farbstoffen am 5'OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich. Es sind auch Nukleotide kommerziell erhaltlich (Amersham Pharmacia), die beispielsweise eine Cy5-Markierung tragen und die in einer PCR-Reaktion eingebaut werden können.

Die komplexen Amplifikationen, die im Stand der Technik beschrieben werden, sind in ihrem Ergebnis gesamthaft allenfalls statistisch kontrollierbar. Nach einer Amplifikation, durchgeführt mit zwei unspezifisch bindenden Primern, ist es praktisch unmöglich Anzahl und Länge der verschiedenen produzierten Fragmente zu bestimmen.

Aufgabe der vorliegenden Erfindung ist es daher, die Nachteile der im Stand der Technik bekannten Verfahren zu überwinden.

Die Aufgabe wird durch ein Verfahren zur kontrollierbaren Durchführung komplexer PCR-Amplifikationen gelöst, wobei man mindestens die folgenden Schritte ausführt:
a) PCR-Amplifikation mit mindestens 50 Primern eines ersten Typs (Typ 1) unterschiedlicher Sequenz, die komplementär zu dem einen Strang einer beliebigen DNA-Probe sind, und die zudem einen Primer oder eine Bibliothek von Primern eines zweiten Typs (Typ 2) enthält, der komplementär zu dem anderen Strang der verwendeten DNA-Probe ist, wobei die Primer des Typs 2 eine erste Markierung (Markierung 1) enthalten
b) Hybridisierung der Amplifikate an einen Oligomer-Array, der Oligonukleotide umfasst, die an die in der PCR Reaktion eingesetzten Primer oder an zu diesen komplementäre Oligonukleotide hybridisieren;
   oder Hybridisierung der Amplifikate an einen Oligomer-Array, der zu den in der PCR Reaktion eingesetzten Primern komplementare Oligomere enthält;
c) Längenbestimmung der an den Array gebundenen Amplifikate durch eine mit der Lange des jeweiligen DNA-Fragments korrelierbare zweite Markierung (Markierung 2), die von der ersten Markierung (Markierung 1) im Schritt a) verschieden ist und
d) Quantifizierung der von Markierung 1 und Markierung 2 stammenden Signale an jedem für die Analyse relevanten Ort des Oligonukleotid-Arrays.

Unter Hybridisierung wird in diesem Zusammenhang eine Zusammenlagerung zweier DNA-Stränge im Sinne der Watson-Crick-Basenpaarungen verstanden, wobei über einen beliebigen Abschnitt von 8 Basen in dem entstandenen Doppelstrang nicht mehr als 25% nicht den Watson-Crick Regeln entsprechende Basenpaarungen auftreten.

Dabei ist vorteilhafterweise erfindungsgemäß bevorzugt, daß man eine PCR-Amplifikation mit mindestens 50 Primern des Typs 1 unterschiedlicher Sequenz, die komplementär zu dem (+)-Strang einer beliebigen DNA-Probe sind, durchführt und die zudem einen Primer oder eine Bibliothek von Primern des Typs 2 enthält, der komplementär zum (-)-Strang ist, wobei die Primer des Typs 2 eine Markierung 1 enthalten.

Gleichermaßen erfindungsgemäß bevorzugt ist es, daß man eine PCR-Amplifikation mit mindestens 50 Primern des Typs 1 unterschiedlicher Sequenz, die komplementär zu dem (-)-Strang sind, durchfuhrt und die zudem einen Primer oder eine Bibliothek von Primern des Typs 2 enthält, der komplementär zum (+)-Strang ist, wobei die Primer des Typs 2 eine Markierung 1 enthalten.

Besonders bevorzugt ist es, daß man die mindestens 50 eingesetzten Primer des Typs 1 derart auswählt, daß diese an die zu amplifizierende DNA spezifisch hybridisieren, wahrend man den zum Gegenstrang komplementären Primer des Typs 2 derart auswählt, daß dieser nicht spezifisch hybridisiert.

Dabei ist es erfindungsgemäß bevorzugt, daß der Primer des Gegenstrangs weniger als 15 Basen umfaßt. Ganz besonders bevorzugt ist hierbei, daß der Primer des Gegenstrangs weniger als 12 Basen umfaßt.

Ferner ist erfindungsgemäß bevorzugt, daß der Primer des Gegenstrangs Positionen mit universellen Basen oder degenerierte Positionen umfaßt.

Es ist weiterhin erfindungsgemaß bevorzugt, daß man die Primer des Gegenstrangs durch kombinatorische Synthese herstellt.

Bevorzugt ist es bei dem erfindungsgemäßen Verfahren, daß Markierung 1 und Markierung 2 Fluoreszenzmarkierungen sind.

Es ist insbesondere bevorzugt, daß Markierung 1 und Markierung 2 Fluoreszenzmarkierungen und/oder ablosbare, in einem Massenspektrometer nachweisbare Massenlabel sind.

Dabei ist besonders bevorzugt, daß man das Verhältnis zwischen den von Markierung 1 und Markierung 2 ausgehenden Signalen an jedem für die Analyse relevanten Ort des Arrays bestimmt.

Erfindungsgemäß bevorzugt ist auch, daß die verwendeten Primer des Typs 1 entweder nur die Basen T, A und C oder aber die Basen T, A, und G enthalten.

Außerdem ist erfindungsgemäß bevorzugt, daß man die DNA vor der Amplifikation chemisch derart behandelt, daß 5-Methylcytosin und Cytosin unterschiedlich reagieren und sich durch die Behandlung eine Veränderung im Basenpaarungsverhalten einer dieser Basen ergibt.

Dabei ist ganz besonders bevorzugt, daß man die Behandlung der DNA vor der Amplifikation mittels einer Bisulfitlösung (=Disulfit, Hydrogensulfit) durchführt.

In dieser Erfindung wird somit eine komplexe Amplifikation beschrieben, welche so geartet ist, daß die Nachteile des Standes der Technik umgangen werden.

Gegenstand der vorliegenden Erfindung ist ein Verfahren zur kontrollierbaren Durchführung komplexer PCR-Amplifikationen. Dazu wird zuerst eine PCR Amplifikation mit mindestens 50 Primern des Typs 1 und einem Primer oder einer Primerbibliothek des Typs 2 durchgeführt, wobei die mindestens 50 Primer des Typs 1 alle hinsichtlich ihrer Sequenz unterschiedlich sind und alle zudem komplementär zu dem (+)-Strang einer beliebigen, unter Verwendung dieses Verfahrens untersuchten DNA-Probe sind. Zudem sind die Primer des Typs oder die Bibliothek von Primern des Typs 2 komplementär zum (-)-Strang der unter Verwendung dieses Verfahrens untersuchten DNA-Probe. Alternativ sind die Primer des Typs 1 komplementär zum (-)-Strang und die des Typs 2 komplementär zum (+)-Strang. Die Primer des Typs 2 enthalten eine Markierung des Typs 1.

In einer besonders bevorzugten Variante des Verfahrens hybridisieren die mindestens 50 eingesetzten Primer des Typs 1 an die zu amplifizierende DNA spezifisch, während der zum Gegenstrang komplementäre Primer des Typs 2 nicht spezifisch hybridisiert, d. h. er kann unter den gewählten Reaktionsbedingungen mehreren Orten der zu untersuchenden DNA-Probe binden. In einer besonders bevorzugten Variante des Verfahrens enthält des Primer des Typs 2 weniger als 15 Basen. In einer bevorzugten Variante des Verfahrens enthält der Primer des Typs 2 weniger als 12 Basen.

Der Primer des Typs 2 kann bevorzugt auch degenerierte Basenpositionen enthalten, d. h. Positionen, die in vergleichbarer Weise an verschiedene Basen des Gegenstrangs binden können, z. B. an A, G oder T. Es können dabei auch bevorzugt sogenannte universelle Basen zum Einsatz kommen, die sowohl an komplementäre T, C, G, und A-Nukleobasen binden können. In einer besonders bevorzugten Variante des Verfahrens wird der Primer des Typs 2 durch kombinatorische Synthese hergestellt, so daß eine Bibliothek von Primern gleicher Länge erhalten wird. Demzufolge werden Bibliotheken von Primer verwendet, die an bestimmten Positionen in einer gegebenen Sequenz unterschiedliche Basen beinhaltet.

In einer bevorzugten Variante des Verfahrens ist die Markierung 1 der Primer des Typs 2 eine Fluoreszenzmarkierung. In einer weiteren besonders bevorzugten Variante des Verfahrens ist die Markierung der Primer des Typs 2 eine Massenmarkierung, die zum Nachweis dieses Primers in einem Massenspektrometer verwendet werden kann. In einer besonders bevorzugten Ausführung erfolgt der Nachweis in einem MALDI-Massenspektrometer, wobei die Ablösung der Massenmarkierung entweder durch Belichtung mit dem Laser des Massenspektrometers oder aber durch den Kontakt mit der MALDI-Matrix durchgeführt wird.

In einer weiteren, besonders bevorzugten Variante des Verfahrens enthalten die verwendeten Primer des Typs 1 entweder nur die Basen T, A und C oder aber die Basen T, A, und G.

Im zweiten Schritt wird eine Hybridisierung der Amplifikate an einen Oligomer-Array, der Oligonukleotide umfasst, die an die in der PCR Reaktion eingesetzten Primer oder an zu diesen komplementäre Oligonukleotide hybridisieren, durchgeführt. Die Oligomere des Arrays sind bevorzugt Oligonukleotide oder PNA-Oligomere. Sie hybridisieren normalerweise nur an einen Strang eines Amplifikates, bevorzugt an den Strang, der die Markierung 1 enthält und demnach einen der Primer des Typs 2 umfasst.

Alternativ wird im zweiten Schritt des Verfahrens eine Hybridisierung der Amplifikate an einen Oligomer-Array, der zu den in der PCR Reaktion eingesetzten Primern komplementäre Oligomere enthält, durchgeführt.

Im dritten Schritt des Verfahrens wird nun eine Längenbestimmung der an jeden für die Analyse relevanten Ort des Arrays gebundenen Amplifikate durch eine mit der Länge des jeweiligen DNA-Fragments korrelierbare Markierung 2, die von der Markierung 1 des ersten Verfahrensschrittes verschieden ist, durchgeführt. In einer bevorzugten Variante des Verfahrens ist die Markierung 2 eine Fluoreszenzmarkierung. In einer weiteren besonders bevorzugten Variante des Verfahrens ist die Markierung der Primer des Typs 2 eine Massenmarkierung, die zum Nachweis dieses Primers in einem Massenspektrometer verwendet werden kann. In einer besonders bevorzugten Ausführung erfolgt der Nachweis in einem MALDI-MAssenspektrometer, wobei die Ablosung der Massenmarkierung entweder durch Belichtung mit dem Laser des Massenspektrometers oder aber durch den Kontakt mit der MALDI-Matrix durchgeführt wird. Die Markierung 2 wird in Abhängigkeit von der Länge des jeweiligen Amplifikates entweder an dieses gebunden oder bei der Amplifikation in dieses integriert. Dies kann bevorzugt durch die Verwendung von fluoreszenz- oder massenmarkierten Triphosphaten in der Amplifikation erfolgen. In einer weiteren, besonders bevorzugten Variante des Verfahrens erfolgt die Längenbestimmung durch Hybridisierung nicht spezifisch bindender Oligonukleotide oder Peptide Nucleic Acids, welche wiederum eine Fluoreszenzmarkierung und/oder Massenmarkierung tragen. Die Fluoreszenzmarkierungen und Massenmarkierungen müssen von denen der Primer des Typs 2 verschieden sein.

Im vierten Schritt des erfindungsgemäßen Verfahrens erfolgt die Quantifizierung der von Markierung 1 und Markierung 2 stammenden Signale an jedem für die Analyse relevanten Ort des Oligonukleotid-Arrays. In einer besonders bevorzugten Variante des Verfahrens wird das Verhältnis der Intensitäten von der Markierung 1 und der Markierung 2 jeweils ausgehenden Signale an jedem für die Analyse relevanten Ort des Arrays bestimmt. In einer bevorzugten Variante des Verfahrens wird damit ermittelt, welche Primer des Typs 1 tatsachlich zu einer Amplifikation beigetragen haben und außerdem wird ermittelt, wie die durchschnittliche Länge der von einem der Primer ausgehenden Amplifikate ist.

In einer besonders bevorzugten Variante des Verfahrens wird die DNA-Probe vor der Amplifikation chemisch derart behandelt, daß 5-Methylcytosin und Cytosin unterschiedlich reagieren und sich durch die Behandlung eine Veränderung im Basenpaarungsverhalten einer dieser Basen ergibt. In einer besonders bevorzugten Variante des Verfahrens wird dies durch eine Behandlung der DNA vor der Amplifikation mit einer Bisulfitlösung (=Disulfit, Hydrogensulfit) erreicht. Bei Verwendung dieser Varianten dient das Verfahren zur kontrollierbaren Herstellung komplexer DNA-Amplifikate, die für die Untersuchung von Cytosin-Methylierungsmustern in der jeweiligen DNA-Probe verwendet werden.

Die folgenden Beispiele erläutern die Erfindung.

### Beispiel 1:

### Komplexe Amplifikation mit 50 Forward-Primern und einem Reverse-Primer und deren Kontrolle

Die Aufgabe, kontrollierte komplexe Amplifikationen durchzuführen, wird durch zwei Verfahrensbestandteile gewährleistet:
1. Zum einen wird die Amplifikation so durchgeführt, dass es zwei Sorten von Primern gibt. Im einfachsten Fall des im Hauptanspruch beschriebenen Verfahrens werden 50 Primer unterschiedlicher Sequenz verwendet, die an den einen Strang des Templates binden, sowie ein Primer, der an den entsprechenden Gegenstrang bindet. Es sollen demnach Amplifikate hergestellt werden, bei denen immer einer der 50 Primer des Typs 1 und auch der Primer des Typs 2 Bestandteil des doppelsträngigen Produktes sind. Um dies zu ermöglichen, müssen die Primer des Typs 2 statistisch wesentlich haufiger an das Templat hybridisieren als die des Typs 1. Zum Beispiel binden die Primer des Typs 1 spezifisch, nicht aber die des Typs 2, die entweder sehr kurz sein können oder aber bevorzugt sogenannte Wobble-Positionen (d. h. Positionen, an denen sich unetrschiedliche Basen befinden können) oder aber degenerierte Basen enthalten können, wobei letztere in DNA nicht natürlich vorkommende Basen sind, welche unspezifische Basenpaarungen mit A, C, T und G oder einer grossen Teilmenge dieser Basen eingehen können.
   Besonders vorteilhaft ist diese Art der Amplifikation in dem Fall, dass die Basenzusammensetzung von (+)- und (-)-Strang des doppelsträngigen Templates sich wesentlich unterscheiden, da nur dann gewährleistet ist, dass die Primer des Typs 2 nur an einen Strang hybridisieren können und demnach nur mit den Primern des Typs 1 und nicht mit weiteren viel häufiger hybridisierenden Primern des Typs 2 Amplifikate bilden können.
   Diese Unterschiedlichkeit der Basenzusammensetzung ist beispielsweise der Fall, wenn genomische DNA mit Natriumbisulfit behandelt wurde. In diesem Fall besteht der behandelte Strang fast ausschliesslich aus den Basen A, T und G, der komplementäre Strang jedoch fast ausschliesslich aus A, T und C. Dies kann beim Design der Primersequenzen sehr vorteilhaft berücksichtigt werden.
2. Das so durchgeführte Verfahren hat den Vorteil, dass nach der Amplifikation nunmehr kontrolliert werden kann, ob die komplexe PCR für jeden der eingesetzten Primer ein Produkt ergeben hat. Dazu werden die Amplifikate an einen Oligomerarray hybridisiert, der zu den Primern des Typs 1 (mindestens 50 verschiedene) komplementäre oder besonders bevorzugt sequenzhomologe Sonden enthält. Im einfachsten Fall werden die 50 eingesetzten Primer auf einer Oberfläche immobilisiert und das komplexe Amplifikat daran hybridisiert. Da jedoch nur die Primer des Typs 2 eine Markierung tragen, werden an der Oberfläche durch Hybridisierung nur die Positionen mit einer Markierung versehen, auf denen sich die Primer befinden, die auch in der Amplifikation ein Produkt ergeben haben. Damit kann leicht identifiziert werden, welche Teile der komplexen Amplifikation erfolgreich waren und es läßt sich auch leicht eine Qualitätskontrolle durchführen.
   Um auch zudem in der Lage zu sein, die korrekte Länge der jeweils synthetisierten Amplifikate zu bestimmen, kann in der Amplifikation eine weitere Markierung eingebaut werden. Im einfachsten Fall ist dies die Lange des Produktes selbst in Form des Molekulargewichts, welches dann beispielsweise durch Massenspektrometrie nachweisbar ist. Bevorzugt jedoch würde man die Markierung während der PCR durch Einbau markierter Nukleotide einführen, beispielsweise durch ein Cy3-gelabeltes dCTP. Da bekannt ist, wieviele Cytosinbasen sich in dem Amplifikat befinden und auch bekannt ist, wieviele gelabeltes dCTP im Vergleich zu dem ebenfalls vorhandenen ungelabelten dCTP eingebaut wird, läßt sich die Länge des Amplifikates berechnen, zumindest aber läßt sich die Reproduzierbarkeit der Amplifikation derart nachweisen. Für die Berechnung ist eine Normierung erforderlich, die den unterschiedlichen Mengen an gebundenem Amplifikat Rechnung tragt, jedoch ist diese sehr leicht möglich, da ja die meßbare Anzahl der Markierungen 1 am Primer des Typs 2 linear mit dem gebundenen Amplifikat korreliert.

### Beispiel 2:

### Durchführung einer komplexen PCR

Genomische DNA wird in Bisulfit-behandelte DNA umgewandelt (Olek et al., Nucl. Acids. Res. 1996, 24, 5064-5066; Stand der Technik). In diesem Beispiel wird die komplexe Amplifikation mit 64 Primeroligonukleotiden des Typs 1 und 4 Primeroligonukleotiden des Typs 2 durchgeführt. Die Primeroligonukleotide des Typs 2 sind mit dem Fluoreszenzfarbstoff Cy5 markiert. Dabei werden die folgenden Primersequenzen verwendet (nur der spezifische Teil ist aufgeführt, zum Abgleich der Annealingtemperatur wird mit unspezifisch bindenden Positionen ("Wobbles") aufgefüllt:
Typ 1: 
TAGTTAGTGTTTAGG
ATTTATTTATTTTTT
ATTTAGATTTTATTG
GTTTTTGTATTTAAG
AGTAGAGTTGAGAAG
GTTTAGAGTAGGGTA
TTTATTATGATTTTG
GTATTATTGTGTTTG
TGGTTAATAGTAATG
TAGTAATTGTATTGG
TTTAGTATTTTGTTG
TAAGTTTTATGAGGT
GATTTGTTTTAGGTA
 
TGATTAATATGGTGA
GAATAGAAATTAGGG
TATAAGGTTAGGAGA
TTAGAGGTTTTTGAG
ATTTTTGTTTGTAGA
GTTGGATAGAAGAGT
TAAAAAATTAGTTGG
GTATGGTGGTTAGAT
TAATGTAGGTTAGGA
AAGTGGTTAGGTATG
GAATTGAGGTAATGT
TAATTTGAGGTTAGG
TAAAGGAAAGTAGGT
TTTGGAAAAATATAG
AATTAATATGGAAAG
GAGTAATGGAAAGAG
TATTTAAGAGGTTGA
GAGTTGGGTATTAGT
TTTAGAGAGGTGAAG
GTTTTAGATTTGTTG
GTTTTATGAGAGGTT
TTGTATTTGGATAGA
GAAAGTTGTTGTTGT
TGAATTTGTTTTTG
GGTTAGTAGGGTTAT
GTTTTAGGTAGAGGA
TTAGGGAAGTATTAT
GTTTGGTGGTTA
GAGAGGTTATATTTG
GGTTAGTTAGGTTAT
AGTAAATATTGAAAG
TTGAGTTTTTTATAG
TTGAATTTTTATTTA
TGATATTAATTTGTT
TTTGGTTATTAGAGT
ATTTAGTTTATTTGT
 
TAGGTTATTTTTGTA
TATAGAGTATTGGTG
TTAGATTTTAATAGG
GGTTAGTTATGTATT
TAGTAGGTTGTTTAG
AATTTTAGGTTGTT
GTGAGGATATTTATT
GTTATAAGTTAAGGA
TTTATTATAGAGGAG
TTGTGGTTTGGA
TGATTTTAGATGTAG
GAGGTTAAAAGGT
TTAAGGTATATTAGG
TTTGAAGTAATTGTA
GTTGTATTGGATAGT
Typ 2:

ACACTCCAACCT
ACCTCAACCTCC
AAAACAAAACCC
AAAACAAAACCA

Die PCR wird mit folgendem Reaktionsansatz durchgeführt:
Reaktionsansatz komplex PCR PCR (25 µl)
1 µl Hydrogensulfit behandelte DNA
2,5 µl PCR buffer (10x, Qiagen)
1 µl Mischung der Typs Primer (64 Primeroligonukleotide, 0,78 pmol/µl von jedem)
1 µl Mischung der Typ2 Primer (4 Primeroligonukleotide, Cy5-markiert, 12,48 pmol/µl von jedem)
0,8 µl dNTPs (25 mM pro dNTP, Gibco-BRL)
3 µl MgCl₂ (15 mM )
15,5 µl Wasser (für die Molekularbiologie, Fluka)
0,2 µl Polymerase (1 unit) (HotstarTaq, Qiagen)

Die PCR-Reaktion wird im Master Cycler Gradient (Eppendorf, Hamburg) mit folgendem Programm durchgeführt.
15 min 95 °C
60 s 95 °C
45 s 35 °C x 39
120 s 65 °C
10 min 65 °C
Halt bei 4 °C

Die erzeugten PCR-Amplifikate werden durch Agarosegel-Elektrophorese (1,5 % Agarose in 0,5xTBE-Puffer, Sambrook et al.) analysiert. Hierfur werden 4 µl des PCR-Ansatzes der Gelelektrophorese unterzogen. Unter den angegeben Bedingungen werden 64 DNA-Fragmente mit den nachfolgend angegebenen Primerpaaren gleichzeitig erfolgreich amplifiziert.

### Beispiel 3:

### Herstellung einer unbekannt methylierten DNA Probe mittels Mulciplex-PCR

Das folgende Beispiel bezieht sich auf die Herstellung einer unbekannt methylierten DNA- Probe, die mit der heruntermethylierten Referenz DNA aus Beispiel 2 verglichen wird. Man setzt eine genomische DNA-Probe ein, die in diesem Fall mit dem Restriktionsenzym MssI gespalten wurde. Die Probe wird anschließend mit Hydrogensulfit (= Bisulfit, Disulfit) umgesetzt. Dabei kann man nach 2 verschiedenen Methoden vorgehen. Die erste Methode (Olek et al., Nucl. Acids. Res. 1996, 24, 5064-5066) ist eine Umsetzung mit Hydrogensulfit und einem Radikalfänger, wobei die DNA in Agarose eingebettet ist. Die Desulfonierung der DNA erfolgt ebenfalls in Agarose. Die DNA wird in diesem Fall ohne weitere Reinigungsopperationen in eine Preamplifikation (PEP = primer exiension preamplification) eingesetzt. Alternativ wird die DNA ohne Agarosematrix ebenfalls mit Hydrogensulfit (= Bisulfit, Disulfit) und einem Radikalfänger bei erhöhter Temperatur chemisch umgewandelt. Ein organisches Reagenz, welches die Denaturierung unterstützt, wird zugesetzt und der Ansatz bei erhohter Temperatur inkubiert. Durch die Behandlung mit Hydrogensulfit werden in beiden Methoden alle Cytosin-Basen zu Uracil umgesetzt wobei Methylcytosine erhalten bleiben. Zur Reinigung der ohne Agarosematrix mit Bisulfit behandelten DNA wird diese auf eine Reversed Phase C18 Festphase gebunden und durch Waschen mit einer geeigneten Pufferlösung von Chemikalien befreit. Anschließend wird die DNA mit einem polaren Lösungsmittel wie z. B. Acetonitril und Wasser eluiert und auf ein kleineres Volumen konzentriert. Die Präamplifikation der mit Hydrogensulfit behandelten DNA wird mit sehr kurzen Primeroligonukleotiden (5'-TTATAATGTTTT und 5'-TAATATACTAAT) durchgeführt.

Reaktionsansatz (20 µl):
1 µl Bisulphit-DNA (0,2-1 ng)
2 µl Reaktions-Puffer (10x, Qiagen)
2 µl dNTP's (10mM pro dNTP, Fermentas)
1 µl Primer (TTATAATGTTTT) 25 pmol
1 µl Primer (TAATATACTAAT) 25 pmol
0,2 µl Polymerase (1 unit) (HotstarTaq, Qiagen)
12,8 µl Wasser (für die Molekularbiologie, Fluka)

Die nachfolgende Amplifikation mit Cy5 gelabelten Primeroligonukleotiden erfolgt mit den im Beispiel 2 beschriebenen Primeroligonukleotiden, wobei das gleiche Primeroligonukleotid Cy5 gelabelt ist. Die Amplifikate werden zur Analyse ebenfalls einer Agarosegel-Elektrophorese unterzogen.

### Beispiel 4: Vergleich der unbekannt methylierten DNA Probe mit der heruntermethylierten Referenz-DNA

Der Vergleich der unbekannt methylierten DNA Probe mit der runtermethylierten Referenz DNA erfolgt vorzugsweise durch Hybridisierung auf ein Oligonukleotidarray. Entsprechend der Position auf dem Array ist sind fluoreszierende Punkte sichtbar. Es fällt auf, dass bestimmte Punkte auf dem Array relativ zu den anderen und zur Referenz-DNA eine deutlich erhöhte oder verminderte Fluoreszenz zeigen, sofern die Amplifikate in den einzelnen zu untersuchenden Proben in vergleichbarer Konzentration vorliegen. Es werden die Intensitäten des Fluorszenzfarbstoffes Cy5 (635 nm) in den einzelnen Amplifikaten gemessen. Die Art und Weise der Auswertung von Fluoreszenzmessungen sind dem Fachmann bekannt.

## Patentansprüche

1. Verfahren zur kontrollierbaren Durchführung komplexer PCR-Amplifikationen, wobei man mindestens die folgenden Schritte ausführt:
a) PCR-Amplifikation mit mindestens 50 Primern eines ersten Typs (Typ 1) unterschiedlicher Sequenz, die komplementär zu dem einen Strang einer beliebigen DNA-Probe sind, und in den zudem einen Primer oder eine Bibliothek von Primern eines zweiten Typs (Typ 2) verwendet wird, der komplementär zu dem anderen Strang der verwendeten DNA-Probe ist, wobei die Primer des Typs 2 eine erste Markierung (Markierung 1) enthalten
b) Hybridisierung der Amplifikate an einen Oligomer-Array, der Oligonukleotide umfasst, die an die in der PCR Reaktion eingesetzten Primer oder an zu diesen komplementare Oligonukleotide hybridisieren;
oder Hybridisierung der Amplifikate an einen Oligomer-Array, der zu den in der PCR Reaktion eingesetzten Primern komplementare Oligomere enthält;
c) Längenbestimmung der an den Array gebundenen Amplifikate durch eine mit der Länge des jeweiligen DNA-Fragments korrelierbare zweite Markierung (Markierung 2), die von der ersten Markierung (Markierung 1) im Schritt a) verschieden ist und
d) Quantifizierung der von Markierung 1 und Markierung 2 stammenden Signale an jedem für die Analyse relevanten Ort des Oligonukleotid-Arrays.

2. Verfahren gemäß Anspruch 1, **dadurch gekennzeichnet, daß** man eine PCR-Amplifikation mit mindestens 50 Primern des Typs 1 unterschiedlicher Sequenz, die komplementär zu dem (+)-Strang einer beliebigen DNA-Probe sind, durchführt und die zudem einen Primer oder eine Bibliothek von Primern des Typs 2 enthält, der komplementär zum (-)-Strang ist, wobei die Primer des Typs 2 eine Markierung 1 enthalten.

3. Verfahren nach Anspruch 1, **dadurch gekennzeichnet, daß** man eine PCR-Amplifikation mit mindestens 50 Primern des Typs 1 unterschiedlicher Sequenz, die komplementär zu dem (-)-Strang sind, durchführt und die zudem einen Primer oder eine Bibliothek von Primern des Typs 2 enthält, der komplementär zum (+)-Strang ist, wobei die Primer des Typs 2 eine Markierung 1 enthalten.

4. Verfahren nach einem der voranstehenden Ansprüche, **dadurch gekennzeichnet, daß** man die mindestens 50 eingesetzten Primer des Typs 1 derart auswählt, daß diese an die zu amplifizierende DNA spezifisch hybridisieren, während man den zum Gegenstrang komplementären Primer des Typs 2 derart auswählt, daß dieser nicht spezifisch hybridisiert.

5. Verfahren nach Anspruch 4, **dadurch gekennzeichnet, daß** der Primer des Gegenstrangs weniger als 15 Basen umfaßt.

6. Verfahren nach Anspruch 4, **dadurch gekennzeichnet, daß** der Primer des Gegenstrangs weniger als 12 Basen umfaßt.

7. Verfahren nach Anspruch 4, **dadurch gekennzeichnet, daß** der Primer des Gegenstrangs Positionen mit universellen Basen oder degenerierte Positionen umfaßt.

8. Verfahren nach Anspruch 4, **dadurch gekennzeichnet, daß** man die Primer des Gegenstrangs durch kombinatorische Synthese herstellt.

9. Verfahren nach einem der vorangehenden Ansprüche, **dadurch gekennzeichnet, daß** Markierung 1 und Markierung 2 Fluoreszenzmarkierungen sind.

10. Verfahren nach einem der vorangehenden Ansprüche, **dadurch gekennzeichnet, daß** Markierung 1 und Markierung 2 Fluoreszenzmarkierungen und/oder ablösbare, in einem Massenspektrometer nachweisbare Massenlabel sind.

11. Verfahren nach Anspruch 9 oder 10, **dadurch gekennzeichnet, daß** man das Verhältnis zwischen den von Markierung 1 und Markierung 2 ausgehenden Signalen an jedem für die Analyse relevanten Ort des Arrays bestimmt.

12. Verfahren nach einem der vorangehenden Ansprüche, **dadurch gekennzeichnet, daß** die verwendeten Primer des Typs 1 entweder nur die Basen T, A und C oder aber die Basen T, A, und G enthalten.

13. Verfahren nach einem der vorangehenden Ansprüche, **dadurch gekennzeichnet, daß** man die DNA vor der Amplifikation chemisch derart behandelt, daß 5-Methylcytosin und Cytosin unterschiedlich reagieren und sich durch die Behandlung eine Veränderung im Basenpaarungsverhalten einer dieser Basen ergibt.

14. Verfahren nach Anspruch 13, **dadurch gekennzeichnet, daß** man die Behandlung der DNA vor der Amplifikation mittels einer Bisulfitlösung (=Disulfit, Hydrogensulfit) durchführt.

## Claims

1. A method for the controllable conducting of complex PCR amplifications, wherein at least the following steps are conducted:
a) PCR amplification with at least 50 primers of a first type (type 1) of different sequence, which are complementary to one of the strands of a random DNA sample, and in which also a primer or a library of primers of a second type (type 2) is used, which is complementary to the other strand of the DNA sample used, whereby the type-2 primers contain a first label (label 1);
b) hybridizing of the amplified products to an oligomer array, which comprises oligonucleotides that hybridize to the primers utilized in the PCR reaction or to oligonucleotides that are complementary to these;
or hybridizing of the amplified products to an oligomer array, which contains oligomers complementary to the primers utilized in the PCR reaction;
c) length determination of the amplified products bound to the array by a second label (label 2) which can be correlated with the length of the respective DNA fragment, and which is different from the first label (label 1) in step a) and
d) quantification of the signals originating from label 1 and label 2 at each site of the oligonucleotide array relevant for the analysis.

2. The method according to claim 1, further **characterized in that** a PCR amplification is conducted with at least 50 type-1 primers of different sequence, which are complementary to the (+) strand of a random DNA sample, and also with a type-2 primer or a library of type-2 primers, which is complementary to the (-) strand, wherein the type-2 primers contain a label 1.

3. The method according to claim 1, further **characterized in that** a PCR amplification is conducted with at least 50 type-1 primers of different sequence, which are complementary to the (-) strand, and also with a type-2 primer or a library of type-2 primers, which is complementary to the (+) strand, wherein the type-2 primers contain a label 1.

4. The method according to one of the preceding claims, further **characterized in that** the at-least 50 type-1 primers used are selected in such a way that they specifically hybridize to the DNA to be amplified, whereas type-2 primers complementary to the couterstrand are selected in such a way that they do not specifically hybridize.

5. The method according to claim 4, further **characterized in that** the primer of the counterstrand comprises fewer than 15 bases.

6. The method according to claim 4, further **characterized in that** the primer of the counterstrand comprises fewer than 12 bases.

7. The method according to claim 4, further **characterized in that** the primer of the counterstrand comprises positions with universal bases or degenerated positions.

8. The method according to claim 4, further **characterized in that** the primers of the counterstrand are prepared by combinatory synthesis.

9. The method according to one of the preceding claims, further **characterized in that** label 1 and label 2 are fluorescent labels.

10. The method according to one of the preceding claims, further **characterized in that** label 1 and label 2 are fluorescent labels and/or mass labels that can be removed and can be detected in a mass spectrometer.

11. The method according to claim 9 or 10, further **characterized in that** the ratio between the signals proceeding from label 1 and label 2 is determined at each site of the array relevant for the analysis.

12. The method according to one of the preceding claims, further **characterized in that** the type-1 primers used contain either only the bases T, A and C or the bases T, A and G.

13. The method according to one of the preceding claims, further **characterized in that** the DNA is chemically treated prior to the amplification in such a way that 5-methylcytosine and cytosine react differently and a change in base-pairing behavior of one of these bases results due to the treatment.

14. The method according to claim 13, further **characterized in that** the DNA is treated prior to amplification by means of a bisulfite solution (= disulfite, hydrogen sulfite).

## Revendications

1. Procédé pour la réalisation contrôlable d'amplifications PCR complexes, où l'on réalise au moins les étapes suivantes :
a) l'amplification PCR avec au moins 50 amorces d'un premier type (type 1) de séquences différentes, qui sont complémentaires d'un brin d'une sonde d'ADN quelconque, et dans lequel on utilise une amorce ou une banque d'amorces d'un deuxième type (type 2), qui est complémentaire de l'autre brin de la sonde d'ADN utilisée, où l'amorce de type 2 contient un premier marquage (marquage 1) ;
b) l'hybridation du produit d'amplification sur une matrice d'oligomères, qui comprend des oligonucléotides, qui s'hybrident aux amorces mises en oeuvre dans la réaction PCR ou à un oligonucléotide complémentaire de celles-ci ;
ou l'hybridation du produit d'amplification sur une matrice d'oligomères, qui contient des oligomères complémentaires des amorces mises en oeuvre dans la réaction PCR ;
c) la détermination de la longueur du produit d'amplification lié à la matrice, par un deuxième marquage (marquage 2) corrélable à la longueur de chaque fragment d'ADN, qui est différent du premier marquage (marquage 1) de l'étape a), et
d) la quantification des signaux provenant du marquage 1 et du marquage 2 en chaque site important pour l'analyse, de la matrice d'oligonucléotides.

2. Procédé selon la revendication 1, **caractérisé en ce que** l'on réalise une amplification PCR avec au moins 50 amorces de type 1 de séquences différentes, qui sont complémentaires du brin (+) d'une sonde d'ADN quelconque, et qui contient de plus, une amorce ou une banque d'amorces de type 2, qui est complémentaire du brin (-),
où les amorces de type 2 contiennent un marquage 1.

3. Procédé selon la revendication 1, **caractérisé en ce que** l'on réalise une amplification PCR avec au moins 50 amorces de type 1 de séquences différentes, qui sont complémentaires du brin (-), et qui contient de plus, une amorce ou une banque d'amorces de type 2, qui est complémentaire du brin (+), où les amorces de type 2 contiennent un marquage 1.

4. Procédé selon l'une quelconque des revendications précédentes, **caractérisé en ce que** l'on choisit les au moins 50 amorces de type 1 mises en oeuvre, de sorte que celles-ci s'hybrident de manière spécifique sur l'ADN à amplifier, alors que l'on choisit l'amorce de type 2, complémentaire au brin opposé, de sorte que celle-ci ne s'hybride pas de manière spécifique.

5. Procédé selon la revendication 4, **caractérisé en ce que** l'amorce du brin opposé comprend moins de 15 bases.

6. Procédé selon la revendication 4, **caractérisé en ce que** l'amorce du brin opposé comprend moins de 12 bases.

7. Procédé selon la revendication 4, **caractérisé en ce que** l'amorce du brin opposé comprend des positions avec des bases universelles ou des positions dégénérées.

8. Procédé selon la revendication 4, **caractérisé en ce que** l'amorce du brin opposé est préparée par synthèse combinatoire.

9. Procédé selon l'une quelconque des revendications précédentes, **caractérisé en ce que** le marquage 1 et le marquage 2 sont des marquages par fluorescence.

10. Procédé selon l'une quelconque des revendications précédentes, **caractérisé en ce que** le marquage 1 et le marquage 2 sont des marquages par fluorescence et/ou sont des labels de masse éliminables, détectables dans un spectromètre de masse.

11. Procédé selon la revendication 9 ou 10, **caractérisé en ce que** l'on détermine le rapport entre les signaux provenant du marquage 1 et du marquage 2 en chaque site important pour l'analyse de la matrice.

12. Procédé selon l'une quelconque des revendications précédentes, **caractérisé en ce que** les amorces de type 1 utilisées contiennent soit seulement les bases T, A et C soit seulement les bases T, A et G.

13. Procédé selon l'une quelconque des revendications précédentes, **caractérisé en ce que** l'on traite chimiquement l'ADN avant l'amplification, de sorte que la 5-méthylcytosine et la cytosine réagissent différemment et que par le traitement, on obtienne une modification du comportement d'appariement des bases pour une de ces bases.

14. Procédé selon la revendication 13, **caractérisé en ce que** l'on réalise le traitement de l'ADN avant l'amplification à l'aide d'une solution de bisulfite (= disulfite, hydrogénosulfite).
